# EUROPEAN PATENT SPECIFICATION

(11) **EP 0 664 791 B1**
(45) Date of publication and mention of the grant of the patent: **16.12.1998**
(21) Application number: 93920976.3
(22) Date of filing: 21.09.1993
(51) Int. Cl.: C07C 249/12, C07C 249/08, C07C 251/60, A01N 33/24, C07D 333/24, C07D 239/34, C07D 239/38, C07D 213/70

(54) **PROCESS FOR THE PREPARATION OF OXIME DERIVATIVES, CERTAIN INTERMEDIATES AND THE USE OF THE OXIME DERIVATIVES AS FUNGICIDES**
VERFAHREN ZUR HERSTELLUNG VON OXIMDERIVATEN, EINIGE INTERMEDIATE UND DIE VERWENDUNG DER OXIMDERIVATE ALS FUNGIZIDE
PROCEDE DE PREPARATION DE DERIVES D'OXIME, CERTAINS INTERMEDIAIRES ET LEUR UTILISATION EN TANT QUE FONGICIDES

(30) Priority: 14.10.1992 GB 9221584
(43) Date of publication of application: 02.08.1995
(73) Proprietor: ZENECA LIMITED, London W1Y 6LN (GB)
(72) Inventor: WORTHINGTON, Paul Anthony, Maidenhead, Berkshire SL6 8HY (GB); ASPINALL, Ian Henry, Bracknell, Berkshire RG12 2TR (GB)
(74) Representative: Tierney, Francis John
(86) International application number: GB9301979
(87) International publication number: WO9408948

(56) References cited:
- EP-A- 0 414 153
- EP-A- 0 460 575
- EP-A- 0 468 684
- WO-A-90/07493

## Description

The present invention relates to chemical processes for the preparation of oximes and to certain intermediate oximes.

Various compounds of formula (Ia) wherein R² is hydrogen and R³ is hydrogen or methyl are disclosed in EP-A-0370629, WO92/13830, WO92/18487, EP-A-0463488, EP-A-0414153, EP-A-0460575 and WO92/18494 (which was published after the priority date of the present application).

The present invention provides a process for the preparation of a compound of formula (I), wherein Y is 0, S(O)ₙ (wherein n is 0, 1 or 2) or NR⁵; W is CH₃O.CH=C.CO₂CH₃, CH₃O.CH=C.CONR⁶R⁷, CH₃O.N=C.CO₂CH₃ or CH₃O.N=C.CONR⁶R⁷; R¹ is hydrogen, C₁₋₄ alkyl, C₁₋₄ haloalkyl or cyano; R², R³,R⁵, R⁶ and R⁷ are independently hydrogen or C₁₋₄ alkyl; p is 1; and R⁴ is hydrogen, C₁₋₁₀ alkyl, optionally substituted aryl, optionally substituted heteroaryl, optionally substituted arylalkyl or optionally substituted heteroarylalkyl; the process comprising reacting a compound of formula (II), wherein W, R¹, R² and R³ are as previously defined and L is a leaving group, with a compound of formula (III), wherein R⁴ and Y are as previously defined; preferably at a temperature in the range 0-120°C, especially in the range 20-60°C, and in a polar solvent such as acetone, N,N-dimethylformamide or N-methylpyrrolidinone.

In one particular aspect the present invention provides a process for the preparation of a compound of formula (I), wherein R¹, R², R³, R⁴, W, p and Y are as defined above, the process comprising the steps of:
a) dehydroxylating a compound of formula (IV), wherein Y is oxygen and R¹, R², R³ and W are as defined above, for example with methanesulphonyl chloride or p-toluenesulphonyl chloride, in the presence of a convenient base such as pyridine or triethylamine, in the presence of a solvent such as diethyl ether or tetrahydrofuran at a temperature in the range 0-60°C, to form a compound of formula (II), wherein R¹, R², R³, W and L are as defined above; and,
b) reacting the product of (a) with a compound of formula (III), wherein Y is 0, S(O)ₙ or NR⁵ and R⁴, n and R⁵ are as defined above in a solvent such as acetone, N,N-dimethylformamide or N-methylpyrrolidinone at a temperature in the range 0-120°C.

In another aspect the present invention provides a process for the preparation of a compound of formula (I), wherein Y is O, S(O)ₙ or NR⁵ and R¹, R², R³, R⁴, W, p, n and R⁵ are as defined above, the process comprising the steps of:
a) reacting a compound of formula (V), wherein W is as defined above, in a polar solvent such as water, methanol or ethanol at a temperature in the range 0-120°C, with a compound of formula (VI), wherein Y is oxygen and R¹, R² and R³ are as defined above, to form a compound of formula (IV) wherein Y is oxygen;
b) dehydroxylating the compound of formula (IV), wherein Y is oxygen and R¹, R², R³ and W are as defined above, to form a compound of formula (II), wherein R¹, R², R³, W and L are as defined above; and
c) reacting the product of (b) with a compound of formula (III), wherein Y is 0, S(O)ₙ or NR⁵, and R⁴, n and R⁵ are as defined above.

In another aspect the present invention provides a process for the preparation of a compound of formula (II), wherein W, L, R¹, R² and R³ are as defined above, the process comprising dehydroxylating a compound of formula (IV), wherein Y is oxygen and R¹, R², R³ and W are as defined above, preferably using methanesulphonyl chloride or p-toluenesulphonyl chloride as a dehydroxylating reagent with a suitable base such as pyridine or triethylamine in a convenient solvent such as diethyl ether or tetrahydrofuran and at a temperature in the range 0-120°C, especially in the range 20-60°C.

In a further aspect the present invention provides a process for the preparation of a compound of formula (II), wherein W, L, R¹, R² and R³ are as defined above, the process comprising the steps of:
a) reacting a compound of formula (V), wherein W is as defined above, with a compound of formula (VI), wherein Y is oxygen, and R¹, R² and R³ are as defined above, in a solvent such as water, methanol or ethanol and at a temperature in the range 0-120°C, to form a compound of formula (IV), wherein Y is oxygen and R¹, R², R³ and W are as defined above; and,
b) dehydroxylating the compound of formula (IV), wherein Y is oxygen, and W, R¹, R² and R³ are as defined above.

In another aspect the present invention provides a process for the preparation of a compound of formula (II), wherein R¹, R², R³ and W are as defined above and L is chlorine, the process comprising reacting a compound of formula (V), wherein W is as defined above, with a compound of formula (VII), wherein R¹, R² and R³ are as defined above, in a solvent such as water, methanol or ethanol at a temperature in the range 0-120°C.

In another aspect the present invention provides a process for the preparation of a compound of formula (I), wherein Y is O, S(O)ₙ or NR⁵, and W, p, R¹, R², R³, R⁴, R⁵ and n are as defined above, the process comprising reacting a compound of formula (IV), wherein Y is O, S(O)ₙ or NR⁵, and W, R¹, R², R³, R⁵ and n are as defined above, with a compound of formula R⁴L, wherein R⁴ is as defined above and L is a leaving group, preferably at a temperature in the range 0-120°C, especially in the range 20-60°C, and in a polar solvent such as acetone, N,N-dimethylformamide or N-methylpyrrolidinone.

In a further aspect the present invention provides a process for the preparation of a compound of formula (I), wherein Y is O, S(O)ₙ or NR⁵ and W, p, R¹, R², R³, R⁴, n and R⁵ are as defined above, the process comprising the steps of:
a) reacting a compound of formula (V), wherein W is as defined above, with a compound of formula (VI), wherein Y is O, S(O)ₙ or NR⁵, and R¹, R², R³, n and R⁵ are as defined above; and,
(b) reacting the product of (a) with a compound of formula R⁴L, wherein R⁴ is as defined above and L is a leaving group, in a solvent such as acetone, N,N-dimethylformamide or N-methylpyrrolidinone at a temperature in the range 0-120°C in the presence of a convenient organic base (such as pyridine or triethylamine) or an inorganic base (such as potassium carbonate or sodium hydride).

In another aspect the present invention provides a process for the preparation of a compound of formula (I), wherein p is 0 and W, R¹, R², R³ and R⁴ are as defined above, the process comprising coupling a compound of formula (II) with a compound of formula R⁴X, wherein R⁴ is as defined above and X is either a leaving group or a metallated species. The coupling reaction can be carried out by treating the metallated species R⁴X with a compound of formula (II) in the presence of a palladium catalyst such as tetrakis(triphenylphosphine)palladium(O).

In another aspect the present invention provides a process for the preparation of a compound of formula (I), wherein p is 0 and W, R¹, R², R³ and R⁴ are as defined above, the process comprising metallating a compound of formula (II) and coupling this metallated species with a compound of formula R⁴X, wherein R⁴ is as defined above and X is a leaving group. For example the compound of formula (II) is metallated with n-butyllithium in a convenient solvent such as diethyl ether or tetrahydrofuran at a temperature of -70-20°C and reacted with R⁴X in a convenient solvent such as diethyl ether or tetrahydrofuran at a temperature of 0-60°C.

When X is a leaving group it can be a halogen atom (including fluorine, chlorine, bromine and iodine) or CH₃SO₂ or CH₃SO₂O. When X is a metallated species it can be a magnesium halide (chloride, bromide or iodide), a zinc halide (chloride, bromide or iodide) or a lithium species.

Examples of the L group are halogen (including fluorine, bromine and iodine but preferably chlorine), CH₃SO₂ and CH₃SO₂O.

Compounds of formula (I) wherein p is 1 can be prepared by reacting a compound of formula (II) with a compound of formula (III) optionally in the presence of a base (such as potassium carbonate or sodium hydride), optionally in a convenient solvent (such as acetone or N,N dimethylformamide) at a temperature of 20-110°C.

Compounds of formula (II) can be prepared by reacting compounds of formula (VI), wherein Y is oxygen, with an agent such as phosphorus trichloride, phosphorus pentachloride, phosphorus tribromide, phosphorus pentabromide, phosphorus oxychloride, phosphorus oxybromide, methanesulphonyl chloride or para-toluenesulphonyl chloride optionally in the presence of a base (such as triethylamine or pyridine), in a convenient solvent (such as chloroform or methylene chloride) at a temperature of 20-110°C.

Alternatively compounds of formula (I), wherein p is 1 can be prepared by reacting compounds of formula (IV) with compounds of formula R⁴L, optionally in the presence of a base (such as sodium hydride, potassium carbonate or triethylamine), in a convenient solvent (such as toluene or N,N-dimethylformamide) at a temperature of 20-110°C.

Compounds of formula (IV) can be prepared by reacting a compound of formula (VI) with a compound of formula (V), optionally in the presence of a base (such as triethylamine or pyridine), in a convenient solvent (such as methanol, ethanol or water) at a temperature of 20-110°C.

In another aspect the present invention provides a compound of formula (II), wherein W is CH₃O.CH=C.CO₂CH₃, CH₃O.CH=C.CONR⁶R⁷, CH₃O.N=C.CO₂CH₃ or CH₃O.N=C.CONR⁶R⁷; R¹ is hydrogen, C₁₋₄ alkyl, C₁₋₄ haloalkoxy or cyano; R², R³, R⁶ and R⁷ are independently hydrogen or C₁₋₄ alkyl; and L is a leaving group; provided that when L is chlorine, W is CH₃O.CH=C.CO₂CH₃, and R² and R³ are both hydrogen then R¹ is not methyl.

In another aspect the present invention provides the compounds of formula (IV), wherein Y is O, S(O)ₙ (wherein n is 0, 1 or 2) or NR⁵ (preferably oxygen); W is CH₃O.CH=C.CO₂CH₃, CH₃O.CH=C.CONR⁶R⁷, CH₃O.N=C.CO₂CH₃ or CH₃O.N=C.CONR⁶R⁷; R¹ is hydrogen, C₁₋₄ alkyl, C₁₋₄ haloalkoxy or cyano; and R², R³, R⁶ and R⁷ are independently hydrogen or C₁₋₄ alkyl.

The compounds listed in Tables Ia to Ii can be made using the process of the present invention. Th group W of all the compounds in Tables Ia to Ii is the (E)-geometric isomer, so E/E and E/Z mixtures refer to isomers about the oxime bond in the side chain.

**TABLE Ia**

| | | | |
|---|---|---|---|
| Compounds in this Table are of formula (VIII) and have the values of Y and R⁴ given below. | | | |

| Compound No | Y | R⁴ | Isomer Mixture |
|---|---|---|---|
| 1 | O | H | E/E:E/Z |
| 2 | S | thien-2-yl | E/E |
| 3 | S | thien-2-yl | E/E:E/Z |
| 4 | O | pyrimidin-2-yl | E/E:E/Z |
| 5 6 | S S | 4-CH₃-C₆H₄ pyrimidin-2-yl | E/E:E/Z E/E:E/Z |
| 7 | S | pyridin-2-yl | E/E:E/Z |
| 8 | S | 4-hydroxypyrimidin-2-yl | E/E:E/Z |
| 9 | S | pyridin-4-yl | E/E:E/Z |
| 10 | S | benzthiazol-2-yl | E/E:E/Z |
| 11 | O | 2-CH₃O-C₆H₄ | E/E:E/Z |
| 12 | S | 4-methylpyrimidin-2-yl | E/E:E/Z |
| 13 | O | 2-CN-C₆H₄ | E/E |
| 14 | O | 2-CN-C₆H₄ | E/E:E/Z |
| 15 | S | benzimidazol-2-yl | E/E:E/E |
| 16 | S | 1-methyltetrazol-5-yl | E/E:E/Z |
| 17 | S | pyridin-2-yl-N-oxide | E/E:E/Z |
| 18 | S | benzoxazol-2-yl | E/E:E/Z |
| 19 | S | 3-methyl-1,2,4-triazol-5-yl | E/E:E/Z |
| 20 | O | 2-CH₃-C₆H₄ | E/E:E/Z |
| 21 | S | 4-n-propyl-6-ethoxypyrimidin-2-yl | E/E:E/Z |
| 22 | S | 4-phenyl-pyrimidin-2-yl | E/E:E/Z |
| 23 | O | 3-CH₃CH₂O-C₆H₄ | E/E:E/Z |
| 24 | O | 3-CH₃-C₆H₄ | E/E:E/Z |
| 25 | S | 4-C₂H₅O-pyrimidin-2-yl | E/E:E/Z |
| 26 | O | 4-CH₃-C₆H₄ | E/E:E/Z |
| 27 | O | 3-CF₃-C₆H₄ | E/E:E/Z |
| 28 | O | 3,4-di-CH₃O-C₆H₃ | E/E:E/Z |
| 29 | S | 5-CF₃-pyridin-2-yl | E/E:E/Z |
| 30 | S | 1-CH₃-imidazol-2-yl | E/E:E/Z |
| 31 | O | 3-CH₃O-C₆H₄ | E/E:E/Z |
| 32 | NH | 3-CF₃-C₆H₄ | E/E |
| 33 | O | 3-NO₂-C₆H₄ | E/E:E/Z |
| 34 | NH | 3-NO₂-C₆H₄ | E/E |
| 35 | S | C₆H₅CH₂ | E/E |
| 36 | S | C₆H₅CH₂ | E/Z |
| 37 | O | (naphth-1-yl)methyl | E/E:E/Z |
| 38 | O | 2,4-di-F-C₆H₃CH₂ | E/E:E/Z |
| 39 | O | 3-CH₃-C₆H₄CH₂ | E/E:E/Z |
| 40 | S | 3-CH₃-C₆H₄CH₂ | E/E |
| 41 | S | 3-CH₃-C₆H₄CH₂ | E/Z |
| 42 | S | 4-Cl-C₆H₄CH₂ | E/E |
| 43 | S | 4-Cl-C₆H₄CH₂ | E/Z |
| 44 | S | C₆H₅CH₂CH₂ | E/E |
| 45 | S | C₆H₅CH₂CH₂ | E/Z |
| 46 | O | thien-2-yl | E/E |
| 47 | NH | pyrimidin-2-yl | E/E:E/Z |
| 48 | O | pyridin-2-yl | E/E:E/Z |
| 49 | O | pyridin-4-yl | E/E:E/Z |
| 50 | O | benzthiazol-2-yl | E/E:E/Z |
| 51 | S | 2-CH₃O-C₆H₄ | E/E:E/Z |
| 52 | O | 4-CH₃-pyrimidin-2-yl | E/E:E/Z |
| 53 | O | benzimidazol-2-yl | E/E:E/Z |
| 54 | O | 1-CH₃-tetrazol-5-yl | E/E:E/Z |
| 55 | O | benzoxazol-2-yl | E/E:E/Z |
| 56 | O | 3-CH₃-1,2,4-triazol-5-yl | E/E:E/Z |
| 57 | S | 2-CH₃-C₆H₄ | E/E:E/Z |
| 58 | S | 3-C₂H₅O-C₆H₄ | E/E:E/Z |
| 59 | S | 4-C₂H₅O-pyrimidin-2-yl | E/E:E/Z |
| 60 | S | 3-CF₃-C₆H₄ | E/E:E/Z |
| 61 | S | 3,4-di-CH₃O-C₆H₃ | E/E:E/Z |
| 62 | O | 5-CF₃-pyridin-2-yl | E/E:E/Z |
| 63 | S | 3-CH₃O-C₆H₄ | E/E:E/Z |
| 64 | O | 3-CF₃-C₆H₄ | E/E:E/Z |
| 65 | S | 3-CF₃-C₆H₄ | E/E:E/Z |
| 66 | S | 3-NO₂-C₆H₄ | E/E:E/Z |
| 67 | O | C₆H₅CH₂ | E/E |
| 68 | O | C₆H₅CH₂ | E/Z |
| 69 | S | 2,4-di-F-C₆H₃CH₂ | E/E:E/Z |
| 70 | NH | 3-CH₃-C₆H₄CH₂ | E/E:E/Z |
| 71 | O | 4-Cl-C₆H₄CH₂ | E/Z |
| 72 | O | 4-Cl-C₆H₄CH₂ | E/E |
| 73 | O | C₆H₅CH₂CH₂ | E/E |
| 74 | O | C₆H₅CH₂CH₂ | E/Z |
| 75 | NH | C₆H₅CH₂CH₂ | E/E |
| 76 | NH | C₆H₅CH₂CH₂ | E/Z |
| 77 | NH | C₆H₅CH₂ | E/E |
| 78 | NH | C₆H₅CH₂ | E/Z |
| 79 | O | 3-C₆H₅O-C₆H₄ | E/E:E/Z |
| 80 | O | quinolin-8-yl | E/E:E/Z |
| 81 | O | 2-CH₃-C₆H₄CH₂ | E/E:E/Z |
| 82 | O | 3-I-C₆H₄CH₂ | E/E:E/Z |
| 83 | O | 4-CH₃-C₆H₄CH₂ | E/E:E/Z |
| 84 | O | C₆H₅ | E/E |
| 85 | O | C₆H₅ | E/Z |
| 86 | O | C₆H₅ | E/E:E/Z |
| 87 | S | pyrimidin-2-yl | E/E |
| 88 | S | pyrimidin-2-yl | E/Z |
| 89 | NCH₃ | C₆H₅ | E/E |

### TABLE Ib

This Table consists of 88 compounds of formula (Ia) wherein W is CH₃O.CH=C.CO₂CH₃, R¹, R² and R³ are all hydrogen and Y and R⁴ have the values given in Table Ia.

### TABLE Ic

This Table consists of 88 compounds of formula (Ia) wherein W is CH₃O.CH=C.CO₂CH₃, R¹ and R² are both methyl, R³ is hydrogen and Y and R⁴ have the values given in Table Ia.

### TABLE Id

This Table consists of 88 compounds of formula (Ia) wherein W is CH₃O.N=C.CO₂CH₃, R¹ is methyl, R² and R³ are both hydrogen and Y and R⁴ have the values given in Table Ia.

### TABLE Ie

This Table consists of 88 compounds of formula (Ia) wherein W is CH₃O.N=C.CO₂CH₃, R¹, R² and R³ are all hydrogen and Y and R⁴ have the values given in Table Ia.

### TABLE If

This Table consists of 88 compounds of formula (Ia) wherein W is CH₃O.N=C.CO₂CH₃, R¹ and R² are both methyl, R³ is hydrogen and Y and R⁴ have the values given in Table Ia.

### TABLE Ig

This Table consists of 88 compounds of formula (Ia) wherein W is CH₃O.N=C.CONHCH₃, R¹ is methyl, R² and R³ are both hydrogen and Y and R⁴ have the values given in Table Ia.

### TABLE Ih

This Table consists of 88 compounds of formula (Ia) wherein W is CH₃O.N=C.CONHCH₃, R¹, R² and R³ are all hydrogen and Y and R⁴ have the values given in Table Ia.

### TABLE Ii

This Table consists of 88 compounds of formula (Ia) wherein W is CH₃O.N=C.CONHCH₃, R¹ and R² are both methyl, R³ is hydrogen and Y and R⁴ have the values given in Table Ia.

### TABLE II

Table II shows selected proton NMR data for certain compounds in Table Ia to Ii. Chemical shifts are measured in parts per million (ppm) from tetramethylsilane and deuterochloroform was used as solvent throughout.

The following abbreviations are used:
- s = singlet: d = doublet
- t = triplet: m = multiplet
- brs = broad singlet: q = quartet
- dd =: doublet of doublets

| Compound No. (Table No) | Proton NMR DATA (δ) |
|---|---|
| 1(Ia) | Major isomer: 1.83(3H,s); 2.62(1H,t); 3.70(3H,s); 3.82(3H,s); 4.13(2H,d); 5.02(2H,s); 7.11-7.17(1H,m); 7.25-7.35(2H,m); 7.40-7.46(1H,m); 7.58(1H,s) ppm. Minor isomer: 1.94(3H,s); 2.53(1H,t); 3.70(3H,s); 3.82(3H,s); 4.29(2H,d); 4.98(1H,s); 7.11-7.17(1H,m); 7.25-7.35(2H,m); 7.40-7.46(1H,m); 7.58(1H,s) ppm. |
| 2(Ia) | Major isomer: 1.99(3H,s); 3.44(2H,s); 3.68(3H,s); 3.80(3H,s); 4.90(2H,s); 6.92-6.96(1H,m); 7.08-7.16(2H,m); 7.25-7.37(4H,m); 7.55(1H,s) ppm. |
| 3(Ia) | Minor isomer: 1.93(3H,s); 3.64(2H,s); 3.69(3H,s); 3.81(3H,s); 4.87(2H,s); 6.92-6.97(1H,m); 7.08-7.17(2H,m); 7.25-7.37(4H,m); 7.56(1H,s) ppm. |
| 4(Ia) | Major isomer: 2.01(3H,s); 3.69(3H,s); 3.81(3H,s); 4.89(2H,s); 5.06(2H,s); 6.97(1H,t); 7.13-7.18(1H,m); 7.29-7.36(2H,m); 7.43-7.48(1H,m); 7.58(1H,s); 8.54(2H,d); ppm. Minor isomer: 1.97(3H,s); 3.70(3H,s); 3.82(3H,s); 5.04(2H,s); 5.25(2H,s); 6.98(1H,t); 7.13-7.18(1H,m); 7.29-7.36(2H,m); 7.43-7.52(1H,m); 7.59(1H,s); 8.55(2H,d) ppm. |
| 5(Ia) | Isomer mixture: 1.91(3H,s); 1.95(3H,s); 2.31(3H,s); 2.32(3H,s); 3.56(2H,s); 3.67(3H,s); 3.68(3H,s); 3.76(2H,s); 3.78(3H,s); 3.80(3H,s); 4.92(2x2H,s); 7.05(2x2H,d); 7.11-7.18(2x1H,m); 7.22-7.38(2x5H,m); 7.55(1H,s); 7.58(1H,s) ppm. |
| 6(Ia) | Major isomer: 2.04(3H,s); 3.77(3H,s); 3.90(3H,s); 4.11(2H,s); 5.11(2H,s); 7.07(1H,t); 7.20-7.26(1H,m); 7.34-7.42(2H,m); 7.49-7.55(1H,s); 7.67(1H,s); 8.60(2H,d) ppm. Minor isomer: 2.07(3H,s); 3.78(3H,s); 3.91(3H,s); 4.20(2H,s); 5.12(2H,s); 7.07(1H,t); 7.20-7.26(1H,m); 7.34-7.42(2H,m); 7.59-7.64(1H,m); 7.68(1H,s); 8.60(2H,d) ppm. |
| 7(Ia) | Major isomer: 1.95(3H,s); 3.68(3H,s); 3.79(3H,s); 3.97(2H,s); 5.00(2H,s); 6.95-7.01(1H,m); 7.01-7.85(5H,m); 7.57(1H,s); 8.40(1H,d) ppm. |
| | Minor isomer: 2.18(3H,s); 3.69(3H,s); 3.80(3H,s); 3.97(2H,s); 5.04(2H,s); 6.95- 7.01(1H,m) ; 7.01-7.85(5H,m); 7.58(1H,s); 8.40(1H,d) ppm. |
| 8(Ia) | Major isomer: 1.95(3H,s); 3.69(3H,s); 3.82(3H,s); 3.96(2H,s); 5.04(2H,s); 6.22(1H,d); 7.11-7.18(1H,m); 7.25-7.34(2H,m); 7.38-7.49(1H,m); 7.58(1H,s); 7.83(1H,d) ppm. Minor isomer: 1.98(3H,s); 3.70(3H,s); 3.80(3H,s); 4.05(2H,s); 5.05(2H,s); 6.08(1H,d); 7.11-7.18(1H,m); 7.25-7.34(2H,m); 7.38-7.49(1H,m); 7.59(1H,s); 7.70(1H,s) ppm. |
| 9(Ia) | Isomer mixture: 1.95(6H,s); 3.64(3H,s); 3.67(3H,s); 3.67(2H,s); 3.79(3H,s); 3.81(3H,s); 3.86(2H,s); 5.00(2H,s); 5.08(2H,s); 7.08(1H,d); 7.11-7.40(7H,m); 7.14(1H,d); 7.45-7.51(1H,m); 7.57(1H,s); 7.60(1H,s); 8.23-8.29(4H,m) ppm. |
| 10(Ia) | Major isomer: 2.00(3H,s); 3.68(3H,s); 3.80(3H,s); 4.14(2H,s); 5.02(2H,s); 7.11-7.18(1H,m); 7.23-7.61(5H,m); 7.56(1H,s); 7.71-7.78(1H,m); 7.81-7.89(1H,m) ppm. Minor isomer: 2.04(3H,s); 3.70(3H,s); 3.82(3H,s); 4.27(2H,s); 5.05(2H,s); 7.11-7.18(1H,m); 7.23-7.61(5H,m); 7.59(1H,s); 7.71-7.78(1H,m); 7.81-7.89(1H,m) ppm. |
| 11(Ia) | Isomer mixture: 1.95(3H,s); 1.98(3H,s); 3.67(6H,s); 3.79(3H,s); 3.80(3H,s); 3.87(3H,s); 3.88(3H,s); 4.61(2H,s); 4.94(2H,s); 5.03(2H,s); 5.04(2H,s); 6.74-6.97(8H,m); 7.12-7.19(2H,m); 7.27-7.37(4H,m); 7.41-7.49(2H,m); 7.57(1H,s); 7.58(1H,s) ppm. |
| 12(Ia) | Major isomer: 1.96(3H,s); 2.45(3H,s); 3.69(3H,s); 3.80(3H,s); 4.04(2H,s); 5.02(2H,s); 6.84(1H,d); 7.11-7.18(1H,m); 7.26-7.35(2H,m); 7.41-7.47(1H,m); 7.58(1H,s); 8.36(1H,d) ppm. Minor isomer: 1.99(3H,s); 2.45(3H,s); 3.70(3H,s); 3.81(3H,s); 4.13(2H,s); 5.03(2H,s); 6.84(1H,d); 7.11-7.18(1H,m); 7.26-7.35(2H,m); 7.51-7.58(1H,m); 7.59(1H,s); 8.36(1H,d) ppm. |
| 13(Ia) | 2.03(3H,s); 3.77(3H,s); 3.81(3H,s); 4.94(2H,s); 5.05(2H,s); 6.89(1H,d); 7.03(1H,t); 7.15-7.21(1H,m); 7.31-7.38(2H,m); 7.42-7.59(3H,m); 7.59(1H,s) ppm. |
| 14(Ia) | 1.99(3H,s); 3.77(3H,s); 3.80(3H,s); 4.64(2H,s); 5.05(2H,s); 6.95-7.03(2H,m); 7.12-7.19(1H,m); 7.28-7.35(2H,m); 7.38-7.47(2H,m); 7.54-7.58(1H,m); 7.58(1H,s) ppm. |
| 15(Ia) | Major isomer: 2.05(3H,s); 3.68(3H,s); 3.67(3H,s); 3.88(2H,s); 5.07(2H,s); 6.90-7.61(8H,m); 7.58(1H,s); 10.10(1H,brs) ppm. Minor isomer: 2.02(3H,s); 3.66(3H,s); 3.67(3H,s); 3.88(2H,s); 5.13(2H,s); 6.59-6.65(1H,d); 6.90-7.61(7H,m); 7.58(1H,s); 10.10(1H,brs) ppm. |
| 16(Ia) | Isomer mixture: 1.97(3H,s); 2.03(3H,s); 3.79(3H,s); 3.80(3H,s); 3.82(2x3H,s); 3.89(3H,s); 3.91(3H,s); 4.09(2H,s); 4.11(2H,s); 4.99(2H,s); 5.01(2H,s); 7.12-7.18(2H,m); 7.28-7.46(6H,m); 7.57(1H,s); 7.58(1H,s) ppm. |
| 17(Ia) | Isomer mixture: 1.97(3H,s); 1.98(3H,s); 3.60(2H,s); 3.75(2x3H,s); 3.79(2x3H,s); 3.81(2H,s); 5.00(2H,s); 5.08(2H,s); 6.73-7.38(13H,m); 7.47-7.53(1H,m); 7.57(1H,s); 7.59(1H,s); 8.20(2x1H,s) ppm. |
| 18(Ia) | Isomer mixture: 1.99(3H,s); 2.07(3H,s); 3.69(3H,s); 3.70(3H,s); 3.81(3H,s); 3.82(3H,s); 4.12(2H,s); 4.18(2H,s); 5.02(2H,s); 5.05(2H,s); 7.10-7.18(2H,m); 7.20-7.41(12H,m); 7.58(1H,s); 7.58-7.62(2H,m); 7.60(1H,m) ppm. |
| 19(Ia) | Major isomer: 1.79(1H,bs); 1.97(3H,s); 2.39(3H,s); 3.70(3H,s); 3.71(2H,s); 3.82(3H,s); 4.96(2H,s); 7.11-7.18(1H,m); 7.26-7.37(3H,m); 7.60(1H,s) ppm. Minor isomer: 1.79(1H,bs); 1.98(3H,s); 2.29(3H,s); 3.71(3H,s); 3.81(2H,s); 3.82(3H,s); 4.96(2H,s); 7.11-7.18(1H,m); 7.26-7.37(2H,m); 7.40-7.45(1H,m); 7.59(1H,s) ppm. |
| 20(Ia) | Major isomer: 1.98(3H,s); 2.22(3H,s); 3.68(3H,s); 3.80(3H,s); 4.52(2H,s); 5.04(2H,s); 6.82(1H,d); 6.98(1H,d); 7.08-7.18(3H,m); 7.28-7.35(2H,m); 7.41-7.48(1H,m); 7.58(1H,s) ppm. Minor isomer: 1.99(3H,s); 2.23(3H,s); 3.68(3H,s); 3.80(3H,s); 4.85(2H,s); 5.03(2H,s); 6.74(1H,d); 7.04-7.18(4H,m); 7.28-7.35(2H,m); 7.41-7.48(1H,m); 7.58(1H,s) ppm. |
| 21(Ia) | Major isomer: 0.93(3H,t); 1.33(3H,t); 1.70(2H,q); 1.97(3H,s); 2.56(2H,q); 3.68(3H,s); 3.82(3H,s); 4.10(2H,s); 4.36(2H,q); 5.02(2H,s): 6.21(1H,s); 7.1-7.5(4H,m); 7.58(1H,s) ppm. Minor isomer: 0.93(3H,t); 1.33(3H,t); 1.70(2H,q); 1.95(3H,s); 2.56(2H,q); 3.68(3H,s); 3.82(3H,s); 4.00(2H,s); 4.37(2H,q); 5.01(2H,s); 6.22(1H,s); 7.1-7.5(4H,m); 7.56(1H,s) ppm. |
| 22(Ia) | Major isomer: 1.98(3H,s); 3.65(3H,s); 3.79(3H,s); 4.12(2H,s); 5.03(2H,s); 7.1-7.5(7H,m); 7.40(1H,d); 7.56(1H,s); 8.1(2H,m); 8.54(1H,d) ppm. Minor isomer: 2.00(3H,s); 3.68(3H,s); 3.80(3H,s); 4.22(2H,s); 5.16(2H,s); 7.1-7.5(7H,m); 7.40(1H,d); 7.59(1H,s); 8.1(2H,m); 8.54(1H,d) ppm. |
| 23(Ia) | Major isomer: 1.40(3H,t); 1.97(3H,s); 3.68(3H,s); 3.80(3H,s); 3.99(2H,q); 4.50(2H,s); 5.05(2H,s); 6.38-6.52(3H,m); 7.10-7.18(2H,m); 7.26-7.36(2H,m); 7.40-7.48(1H,m); 7.57(1H,s) ppm. Minor isomer: 1.40(3H,t); 1.98(3H,s); 3.68(3H,s); 3.80 (3H,s); 3.99(2H,q); 4.83(2H,s); 5.02(2H,s); 6.38-6.52(3H,m); 7.10-7.18(2H,m); 7.26-7.36(2H,m); 7.40-7.48(1H,m); 7.58(1H,s)ppm. |
| 24(Ia) | Major isomer: 1.97(3H,s); 2.30(3H,s); 3.68(3H,s); 3.80(3H,s); 4.50(2H,s); 5.05(2H,s); 6.69-6.79(3H,s); 7.10-7.18(2H,m); 7.27-7.35(2H,m); 7.40-7.48(1H,m); 7.58(1H,s)ppm. Minor isomer: 1.97(3H,s); 2.30(3H,s); 3.69(3H,s); 3.81(3H,s); 4.06(2H,s); 5.04(2H,s); 6.69-6.79(3H,m); 7.10-7.18(2H,m); 7.27-7.35(2H,m); 7.40-7.48(1H,m); 7.58(1H,s)ppm. |
| 25(Ia) | Major isomer: 1.38(3H,t); 1.97(3H,s); 3.69(3H,s); 3.82(3H,s); 4.40(2H,q); 4.50(2H,s); 5.01(2H,s); 6.38(1H,d); 7.11-7.17(1H,m); 7.26-7.34(2H,m); 7.40-7.46(1H,m); 7.57(1H,s); 8.20(1H,d)ppm. Minor isomer: 1.38(3H,t); 1.99(3H,s); 3.69(3H,s); 3.82(3H,s); 4.10(2H,s); 4.40(2H,q); 5.02(2H,s); 6.38(1H,d); 7.11-7.17(1H,m); 7.26-7.34(2H,m); 7.40-7.46(1H,m); 7.58(1H,s); 8.20(1H,d)ppm. |
| 26(Ia) | Major isomer: 1.95(3H,s); 2.28(3H,s); 3.67(3H,s); 3.79(3H,s); 4.49(2H,s); 5.04(2H,s); 6.80(2H,d); 7.05(2H,d); 7.12-7.18(1H,m); 7.26-7.35(2H,m); 7.40-7.47(1H,m); 7.57(1H,s)ppm. Minor isomer: 1.94(3H,s); 2.28(3H,s); 3.67(3H,s); 3.79(3H,s); 4.81(2H,s); 5.02(2H,s); 6.75(2H,d); 7.05(2H,d); 7.12-7.18(1H,m); 7.26-7.35(2H,m); .7.40-7.47(1H,m); 7.58(1H,s)ppm. |
| 27(Ia) | Major isomer: 1.96(3H,s); 3.68(3H,s); 3.80(3H,s); 4.56(2H,s); 5.05(2H,s); 6.95-7.23(4H,m); 7.29-7.39(3H,m); 7.39-7.48(1H,m); 7.57(1H,s)ppm. Minor isomer: 1.96(3H,s); 3.68(3H,s); 3.80(3H,s); 4.87(2H,s); 5.05(2H,s); 6.95-7.23(4H,m); 7.29-7.39(3H,m); 7.39-7.48(1H,m); 7.58(1H,s)ppm. |
| 28(Ia) | Major isomer: 1.97(3H,s); 3.67(3H,s); 3.80(6H,s); 3.83(3H,s); 4.49(2H,s); 5.05(2H,s); 6.30-6.55(2H,m); 6.74(1H,d); 7.12-7.19(1H,m); 7.28-7.36(2H,m); 7.40-7.47(1H,m); 7.57(1H,s)ppm. Minor isomer: 1.96(3H,s); 3.67(3H,s); 3.80(3H,s); 3.83(6H,s); 4.80(2H,s); 5.03(2H,s); 6.30-6.55(2H,m); 6.71(1H,d); 7.12-7.19(1H,m); 7.28-7.36(2H,m); 7.40-7.47(1H,m); 7.58(1H,s)ppm. |
| 29(Ia) | Major isomer: 1.95(3H,s); 3.78(3H,s); 3.80(3H,s); 3.98(2H,s); 5.01(2H,s); 7.12-7.19(1H,m); 7.23-7.34(3H,m); 7.36-7.42(1H,m); 7.53-7.60(1H,m); 7.57(1H,s); 8.63(1H,brs)ppm. Minor isomer: 1.96(3H,s); 3.79(3H,s); 3.81(3H,s); 4.14(2H,s); 5.05(2H,s); 7.12-7.19(1H,m); 7.23-7.60(6H,m); 8.63(1H,brs)ppm. |
| 30(Ia) | 1.95(3H,s); 3.57(3H,s); 3.69(3H,s); 3.70(2H,s); 3.80(3H,s); 4.93(2H,s); 6.91(1H,s); 7.07(1H,s); 7.11-7.17(1H,m); 7.26-7.42(3H,m); 7.56(1H,s)ppm. |
| 31(Ia) | 1.98(3H,s); 3.76(3H,s); 3.78(3H,s); 3.80(3H,s); 4.51(2H,s); 5.05(2H,s); 6.40-6.55(3H,m); 7.10-7.19(2H,m); 7.29-7.35(2H,m); 7.41-7.48(1H,m); 7.58(1H,s)ppm. |
| 32(Ia) | 1.90(3H,s); 3.69(3H,s); 3.81(3H,s); 3.82(2H,d); 5.05(2H,s); 6.74-6.79(1H,m); 6.85(1H,brs); 6.91-6.96(1H,m); 7.13-7.36(5H,m); 7.43-7.48(1H,m); 7.59(1H,s)ppm. |
| 33(Ia) | Isomer mixture: 1.97(6H,s); 3.67(3H,s); 3.68(3H,s); 4.61(2H,s); 4.91(2H,s); 5.07(4H,s); 7.15-7.50(13H,m); 7.58(1H,s); 7.60(1H,s); 7.80-7.88(3H,m)ppm. |
| 34(Ia) | 1.90(3H,s); 3.69(3H,s); 3.82(3H,s); 3.83(2H,d); 4.87(1H,t); 5.05(2H,s); 6.91(1H,dd); 7.12-7.18(1H,m); 7.27-7.35(3H,m); 7.41-7.47(2H,m); 7.53(1H,dd); 7.59(1H,s)ppm. |
| 35(Ia) | 1.95(3H,s); 3.07(2H,s); 3.52(2H,s); 3.69(3H,s); 3.81(3H,s); 5.03(2H,s); 7.12-7.35(8H,m); 7.43-7.49(1H,m); 7.58(1H,s)ppm. |
| 36(Ia) | 1.95(3H,s); 3.34(2H,s); 3.66(3H,s); 3.67(2H,s); 3.78(3H,s); 5.00(2H,s); 7.12-7.33(8H,m); 7.40-7.45(1H,m); 7.57(1H,s)ppm. |
| 37(Ia) | Major isomer: 1.91(3H,s); 3.67(3H,s); 3.78(3H,s); 4.07(2H,s); 4.90(2H,s); 5.05(2H,s); 7.10-7.18(1H,m); 7.25-7.36(2H,m); 7.36-7.55(5H,m); 7.56(1H,s); 7.76-7.89(2H,m); 8.05-8.11(1H,m)ppm. Minor isomer: 1.95(3H,s); 3.64(3H,s); 3.74(3H,s); 4.05(2H,s); 4.41(2H,s); 4.60(2H,s); 7.10-7.18(1H,m); 7.25-7.36(2H,m); 7.36-7.55(6H,m); 7.56(1H,s); 7.76-7.89(2H,m); 8.05-8.11(1H,m)ppm. |
| 38(Ia) | Major isomer: 1.95(3H,s); 3.67(3H,s); 3.80(3H,s); 4.33(2H,s); 4.49(2H,s); 4.96(2H,s); 6.76-6.90(2H,m); 7.10-7.18(1H,m); 7.28-7.48(4H,m); 7.58(1H,s)ppm. Minor isomer: 1.91(3H,s); 3.67(3H,s); 3.80(3H,s); 4.02(2H,s); 4.45(2H,s); 5.03(2H,s); 6.76-6.90(2H,m); 7.10-7.18(1H,m); 7.28-7.48(4H,m); 7.59(1H,s)ppm. |
| 39(Ia) | Isomer mixture: 1.94(3H,s); 1.98(3H,s); 2.36(6H,s); 3.67(3H,s); 3.68(3H,s); 3.79(3H,s); 3.81(3H,s); 4.02(2H,s); 4.35(2H,s); 4.42(2H,s); 4.47(2H,s); 4.97(2H,s); 5.04(2H,s); 7.06-7.19(8H,m); 7.20-7.37(6H,m); 7.39-7.49(2H,m); 7.57(1H,s); 7.58(1H,s)ppm. |
| 40(Ia) | 1.95(3H,s); 2.31(3H,s); 3.07(2H,s); 3.50(2H,s); 3.68(3H,s); 3.80(3H,s); 5.03(2H,s); 6.98-7.08(3H,m); 7.10-7.19(2H,m); 7.23-7.35(2H,m); 7.43-7.49(1H,m); 7.58(1H,s)ppm. |
| 41(Ia) | 1.94(3H,s); 2.28(3H,s); 3.34(2H,s); 3.62(2H,s); 3.63(3H,s); 3.78(3H,s); 4.99(2H,s); 6.99-7.17(5H,m); 7.23-7.32(2H,m); 7.38-7.44(1H,m); 7.56(1H,s)ppm. |
| 42(Ia) | 1.95(3H,s); 3.00(2H,s); 3.43(2H,s); 3.69(3H,s); 3.81(3H,s); 5.02(2H,s); 7.07(2H,q); 7.12-7.38(5H,m); 7.42-7.49(1H,m); 7.59(1H,s)ppm. |
| 43(Ia) | 1.95(3H,s); 3.30(2H,s); 3.59(2H,s); 3.64(3H,s); 3.78(3H,s); 4.98(2H,s); 7.10-7.19(5H,m); 7.27-7.35(2H,m); 7.37-7.42(1H,m); 7.57(1H,s)ppm. |
| 44(Ia) | 1.95(3H,s); 2.58-2.65(2H,m); 2.78-2.85(2H,m); 3.19(2H,s); 3.67(3H,s); 3.77(3H,s); 5.01(2H,s); 7.10-7.30(8H,m); 7.40-7.47(1H,m); 7.55(1H,s)ppm. |
| 45(Ia) | 1.97(3H,s); 2.68-2.75(2H,m); 2.80-2.88(2H,m); 3.40(2H,s); 3.66(3H,s); 3.77(3H,s); 4.99(2H,s); 7.09-7.31(8H,m); 7.38-7.44(1H,m); 7.55(1H,s)ppm. |
| 79(Ia) | 1.95(3H,s); 3.69(3H,s); 3.81(3H,s); 4.49(2H,s); 5.05(2H,s); 6.55-6.70(3H,m); 6.98-7.08(2H,m); 7.09-7.22(3H,m); 7.27-7.38(4H,m); 7.41-7.47(1H,m); 7.58(1H,s)ppm. |
| 80(Ia) | Isomer mixture: 2.0(3H,s); 2.05(3H,s); 3.65(6H,s); 3.80(6H,s); 4.90(2H,s); 5.11(4H,d); 5.21(2H,s); 7.10-7.21(4H,m); 7.30-7.52(12H,m); 7.59(2H,s); 8.11(1H,s); 8.13(1H,s); 8.91(1H,s); 8.94(1H,s)ppm. |
| 81(Ia) | 1.95(3H,s); 2.35(3H,s); 3.67(3H,s); 3.70(3H,s); 4.03(2H,s); 4.44(2H,s); 5.04(2H,s); 7.11-7.22(4H,m); 7.26-7.30(3H,m); 7.31-7.39(1H,m); 7.57(1H,s)ppm. |
| 82(Ia) | 1.92(3H,s); 3.69(3H,s); 3.81(3H,s); 4.02(2H,s); 4.38(2H,s); 5.06(2H,s); 7.02-7.18(2H,m); 7.22-7.37(3H,m); 7.42-7.48(1H,m); 7.57(1H,s); 7.59-7.70(2H,m)ppm. |
| 83(Ia) | 1.90(3H,s); 2.33(3H,s); 3.64(3H,s); 3.80(3H,s); 3.98(2H,s); 4.39(2H,s); 5.03(2H,s); 7.09-7.19(4H,m); 7.22-7.33(3H,m); 7.40-7.47(1H,m); 7.55(1H,s)ppm. |
| 89(Ia) | 1.81(3H,s); 2.90(3H,s); 3.68(3H,s); 3.80(3H,s); 3.94(2H,s); 5.04(2H,s); 6.70-6.81(3H,m); 7.11-7.38(5H,m); 7.41-7.48(1H,m); 7.58(1H,s) ppm. |
| 12(Ig) | Major isomer: 1.90(3H,s); 2.45(3H,s), 2.90(3H,d); 3.96(3H,s); 3.99(2H,s); 4.98(2H,s); 6.73(1H,q); 6.84(1H,d); 7.16-7.21(1H,m); 7.31-7.46(3H,m); 8.33(1H,d) ppm. Minor isomer: 1.95(3H,s); 2.45(3H,s); 2.92(3H,d); 3.96(3H,s); 4.08(2H,s); 5.00(2H,s); 6.73(1H,q); 6.84(1H,d); 7.16-7.21(1H,m); 7.31-7.46(2H,m); 7.48-7.53(1H,m); 8.34(1H,d) ppm. |
| 36(Ig) | 1.90(3H,s); 2.84(3H,d); 3.29(2H,s); 3.65(2H,s); 3.94(3H,s); 4.94(2H,s); 6.68(1H,q); 7.15-7.45(9H,m) ppm. |
| 84(Ib) | 3.68(3H,s); 3.81(3H,s); 4.81(2H,d); 5.09(2H,s); 6.88(2H,d); 6.95-7.00(2H,m); 7.15-7.19(1H,m); 7.26-7.35(4H,m); 7.41-7.47(1H,m); 7.58(1H,s)ppm. |
| 85(Ib) | 3.68(3H,s); 3.86(3H,s); 4.61(2H,d); 5.04(2H,s); 6.90(2H,d); 6.98(1H,t); 7.14-7.19(1H,m); 7.24-7.36(4H,m); 7.42-7.47(1H,m); 7.57-7.61(1H,m); 7.58(1H,s)ppm. |
| 86(Ic) | 1.48(3H,d); 1.80(3H,s); 3.63(3H,s); 3.75(3H,s); 4.87(1H,q); 5.02(2H,s); 6.89-6.94(3H,m); 7.11-7.25(3H,s); 7.27-7.34(2H,m); 7.37-7.42(1H,m); 7.55(1H,s)ppm. |
| 87(Ic) | 1.58(3H,d); 1.96(3H,s); 3.78(3H,s); 3.80(3H,s); 4.70(1H,q); 5.02(2H,s); 6.95(1H,t); 7.10-7.16(1H,m); 7.25-7.31(2H,m); 7.39-7.45(1H,m); 7.55(1H,s); 8.49(2H,d)ppm. |
| 88(Ic) | 1.59(3H,d); 1.96(3H,s); 3.68(3H,s); 3.81(3H,s); 4.70(1H,q); 5.02(2H,s); 6.96(1H,t); 7.10-7.16(1H,m); 7.25-7.31(2H,m); 7.39-7.45(1H,m); 7.57(1H,s); 8.50(2H,d)ppm. |
| 12(Id) | Major isomer: 1.91(3H,s); 2.45(3H,s); 3.85(3H,s); 4.01(2H,s); 4.05(3H,s); 4.99(2H,s); 6.84(1H,d); 7.15-7.21(1H,m); 7.32-7.48(3H,m); 8.35(1H,d) ppm. |
| | Minor isomer: 1.97(3H,s); 2.45(3H,s); 3.88(4H,s); 4.06(3H,s); 4.09(2H,s); 5.00(2H,s); 6.84(1H,d); 7.15-7.21(1H,m); 7.32-7.48(2H,m); 7.49-7.54(1H,m); 8.35(1H,d) ppm. |
| 35(Id) | 1.90(3H,s); 3.01(2H,s); 3.49(2H,s); 3.86(3H,s); 4.05(3H,s); 5.00(2H,s); 7.15-7.50(9H,m) ppm. |
| 36(Id) | 1.93(3H,s); 3.30(2H,s); 3.64(2H,s); 3.81(3H,s); 4.03(3H,s); 4.96(2H,s); 7.12-7.31(6H,m); 7.31-7.48(3H,m) ppm. |
| 12(Ig) | Major isomer: 1.90(3H,s); 2.45(3H,s), 2.90(3H,d); 3.96(3H,s); 3.99(2H,s); 4.98(2H,s); 6.73(1H,q); 6.84(1H,d); 7.16-7.21(1H,m); 7.31-7.46(3H,m); 8.33(1H,d) ppm. Minor isomer: 1.95(3H,s); 2.45(3H,s); 2.92(3H,d); 3.96(3H,s); 4.08(2H,s); 5.00(2H,s); 6.73(1H,q); 6.84(1H,d); 7.16-7.21(1H,m); 7.31-7.46(2H,m); 7.48-7.53(1H,m); 8.34(1H,d) ppm. |
| 36(Ig) | 1.90(3H,s); 2.84(3H,d); 3.29(2H,s); 3.65(2H,s); 3.94(3H,s); 4.94(2H,s); 6.68(1H,q); 7.15-7.45(9H,m) ppm. |

The following Examples illustrate the invention.

### EXAMPLE 1

This Example illustrates the preparation of (E)-methyl 2-[2-(hydroxymethyl-acetoximinomethyl)phenyl]-3-methoxypropenoate (Compound No. 1 of Table Ia).

(E)-Methyl 2-[phthalimidooxymethylphenyl]-3-methoxypropenoate (1.97g) (prepared as described in Example 4 of EP 0463488) was suspended in methanol (20ml) at room temperature, hydrazine hydrate (0.27g) was added and the resulting solution stirred for 3 hours. The white precipitate which formed was filtered off and the solvent removed to give a white semi-solid. This was diluted with diethyl ether, the white solid filtered off and the filtrate evaporated to give (E)-methyl 2-[2-aminooxymethylphenyl]-3-methoxy-propenoate (1.18g, 93%) as a yellow oil which was used immediately in the next stage without further purification.

A mixture of (E)-methyl 2-[2-aminooxymethylphenyl]-3-methoxypropenoate (1.18g), acetol (0.4g) and pyridine (0.2ml) in methanol (10ml) and water (2ml) was stirred at room temperature for 24 hours. The solution was evaporated to dryness to give a yellow gum which was purified by flash column chromatography, eluted with diethyl ether:n-hexane 2:1, to give the title compound (0.71g, 45%) as a yellow gum. ¹H NMR, see Table II, showed this to be a mixture of isomers.

### EXAMPLE 2

This Example illustrates the preparaton of (E),(E)-methyl 2-[2-(2-cyanophenoxymethyl-acetoximinomethyl)phenyl]-3-methoxypropenoate (Compound No. 13 of Table Ia).

A solution of (E)-methyl-2-[2-(hydroxymethyl-acetoximinomethyl)phenyl]-3-methoxypropenoate (0.51g) (prepared as described in Example 1), p-toluenesulphonyl chloride (1.65g) and triethylamine (1.2ml) in dry tetrahydrofuran (20ml) was stirred at room temperature for 12 hours. The solvent was removed under reduced pressure to leave a yellow-orange oil which was purified by flash column chromatography, eluted with n-hexane:diethyl ether 4:1, to give (E)-methyl 2-[2-(chloromethyl--acetoximinomethyl)phenyl]-3-methoxypropenoate (0.15g, 28%) as a mixture of isomers. ¹H NMR (270 MHz): major isomer - δ1.97(3H,s), 3.69(3H,s), 3.82(3H,s), 4.06(2H,s), 5.03(2H,s), 7.12-7.19(1H,m), 7.28-7.37(2H,m), 7.40-7.47(1H,m), 7.57(1H,s) ppm; minor isomer - δ 2.00(3H,s), 3.69(3H,s), 3.82(3H,s), 4.24(2H,s), 5.02(2H,s), 7.12-7.19(1H,m), 7.28-7.37(2H,m), 7.40-7.47(1H,m), 7.57(1H,s) ppm.

A mixture of (E)-methyl 2-[2-(chloromethyl-acetoximinomethyl)phenyl]-3-methoxypropenoate (0.27g), 2-cyanophenol (0.51g) and potassium carbonate (0.60g) in acetone (7ml) and water (1ml) was heated at 60°C for 5 hours. After cooling to room temperature, the solvent was removed under reduced pressure and the residue dissolved in diethyl ether (200ml). The ethereal extract was washed with 2N NaOH, water and dried. Removal of the solvent gave a brown gum which was purified by flash column chromatography, eluted with n-hexane:diethyl ether 1:1, to give the title compound (0.029g, 9%) as a crystalline solid mp 102°C; ¹H NMR see Table II.

A fraction (0.062g, 18%) was also obtained which was a mixture (1:15) of the title compound and the corresponding (Z), (E) isomer; ¹H NMR see Table II.

### EXAMPLE 3

This Example illustrates the preparation of (E)-methyl 2-[2-(benzoxazol-2-yl(thio)methyl-acetoximinomethyl)phenyl]-3-methoxypropenoate (Compound No. 18 of Table Ia).

A mixture of (E)-methyl-2-[2-(chloromethyl-acetoximinomethyl)phenyl]-3-methoxypropenoate (0.21g) (prepared as described in Example 2), 2-mercaptobenzoxazole (0.5g) and potassium carbonate (0.46g) in acetone (7ml) and water (1ml) was heated at 60°C for 5 hours. After cooling to room temperature the solvent was removed under reduced presure. The residue was purified by flash column chromatography, eluted with n-hexane:diethyl ether 1:1, to give the title compound (0.18g, 64%) as a gum: ¹H NMR see Table II.

### EXAMPLE 4

This Example illustrates the preparation (E),(E)-methyl 2-[2-(3-nitroanilinomethyl-acetoximinomethyl)phenyl]-3-methoxypropenoate (Compound No. 34 of Table Ia).

A mixture of (E)-methyl-2-[2-(chloromethyl-acetoximinomethyl)phenyl]-3-methoxypropenoate (0.188g, prepared as described in Example 2), 3-nitroaniline (0.416g) and potassium carbonate (0.416g) in acetone (18ml) and water (8ml) was heated at 60°C for 24 hours. After cooling to room temperature the solvent was removed under reduced pressure. The residue was purified by flash column chromatography, silica eluted with dichloromethane:n-hexane:tetrahydrofuran 20:10:1, to give the title compound (0.022g, 10%) as a yellow gum; ¹H NMR see Table II.

### EXAMPLE 5

This Example illustrates the preparation of (E),(E) and (E),(Z)-methyl 2-[2-(4-chlorobenzyl(thio)methyl-acetoximinomethyl)phenyl]-3-methoxypropenoate (Compound Nos. 42 and 43 in Table Ia).

A solution of (E)-methyl-2-[2-(chloromethyl-acetoximinomethyl)phenyl]-3-methoxypropenoate (0.223g, prepared as described in Example 2) in DMF (6ml) was added dropwise to the sodium salt of 4-chlorobenzylmercaptan (prepared from 4-chlorobenzyl mercaptan (0.113g) and sodium hydride (0.029g)] in DMF (6ml) at 20°C. After complete addition the mixture was stirred for 30 minutes, poured into water and extracted with diethyl ether. The ethereal extracts were washed, dried and the solvent removed to give a crude gum which was purified by flash column chromatography, silica gel eluted with n-hexane: diethyl ether 3:1, to give the title compounds (0.211g, 68%) as separate isomers; ¹H NMR see Table II.

### EXAMPLE 6

This Example illustrates an alternative preparation of (E)-methyl-2-[2-[chloromethyl-acetoximinomethyl)phenyl]-3-methoxypropenoate (for use in Examples 2, 3, 4 and 5).

A mixture of (E)-methyl 2-(2-aminoxymethylphenyl)-3-methoxypropenoate (3.2g, prepared as described the first stage of Example 1), chloroacetone (0.83g), and pyridine (0.6ml) in ethanol (30ml) and water (4ml) was stirred at room temperature for 24 hours. The solution was evaporated to dryness to give a yellow gum which was purified by flash column chromatography, silica gel eluted with diethyl ether : n-hexane 4:1, to give the title compound (2.69g, 64%) as a 1:1 mixture of isomers. ¹H NMR (270 MHz): δ 1.97(3H,s); 2.0(3H,s); 3.68(6H,s); 3.82(6H,s); 4.06(2H,s); 4.24(2H,s); 5.02(2H,s); 5.03(2H,s); 7.12-7.19(2H,m); 7.28-7.37(4H,m); 7.40-7.47(2H,m); 7.57(2H,s) ppm.

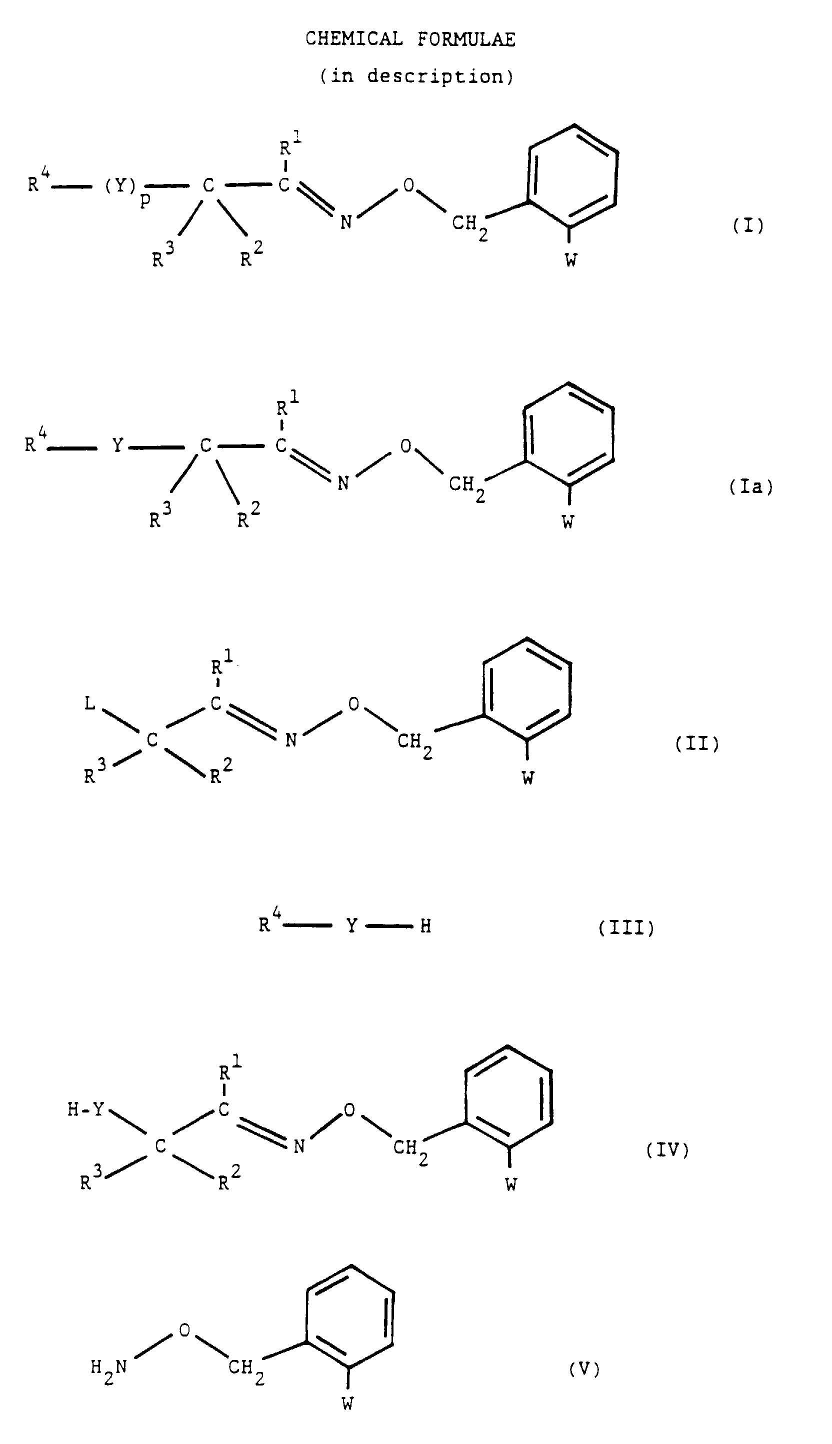

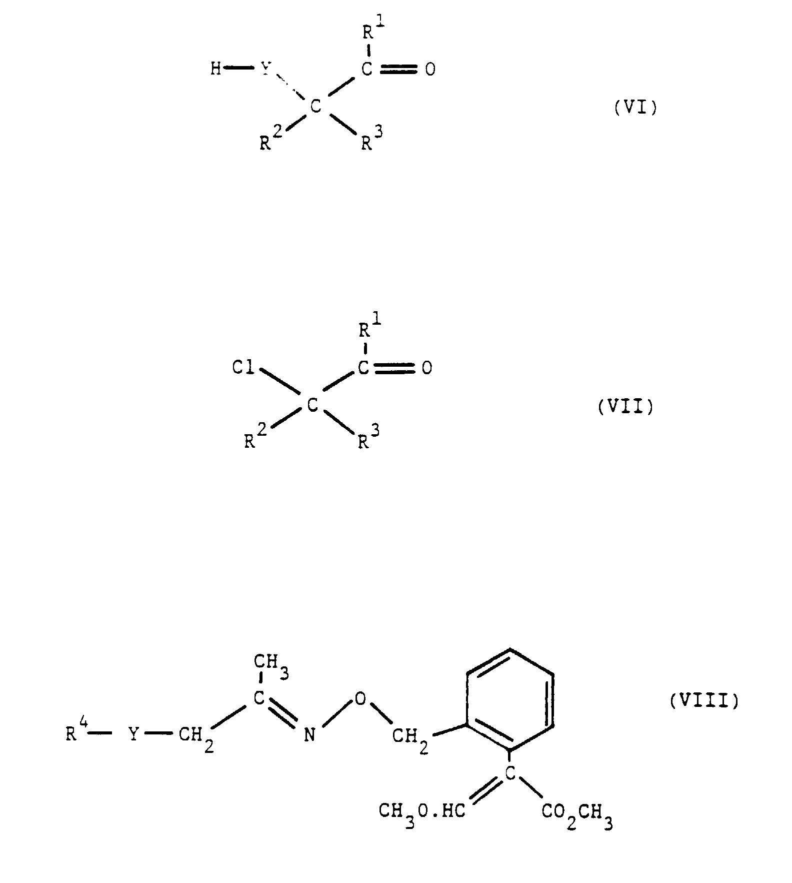

## Claims

1. A process for the preparation of a compound of formula (I): 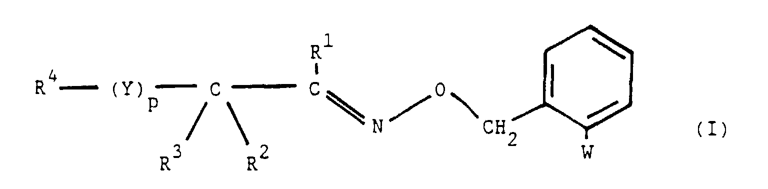 wherein Y is 0, S(O)ₙ (wherein n is 0, 1 or 2) or NR⁵; W is CH₃O.CH=C.CO₂CH₃, CH₃O.CH=C.CONR⁶R⁷, CH₃O.N=C.CO₂CH₃ or CH₃O.N=C.CONR⁶R⁷; R¹ is hydrogen, C₁₋₄ alkyl, C₁₋₄ haloalkyl or cyano; R², R³, R⁵, R⁶ and R⁷ are independently hydrogen or C₁₋₄ alkyl; p is 1; and R⁴ is hydrogen, C₁₋₁₀ alkyl, optionally substituted aryl, optionally substituted heteroaryl, optionally substituted arylalkyl or optionally substituted heteroarylalkyl; the process comprising reacting a compound of formula (II): 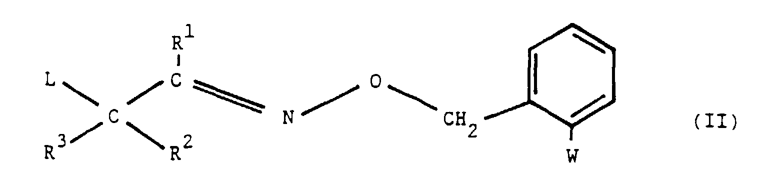 wherein W, R¹, R² and R³ are as previously defined and L is a leaving group, with a compound of formula (III):
R⁴―Y―H (III)
wherein R⁴ and Y are as previously defined.

2. A process for the preparation of a compound of formula (II): 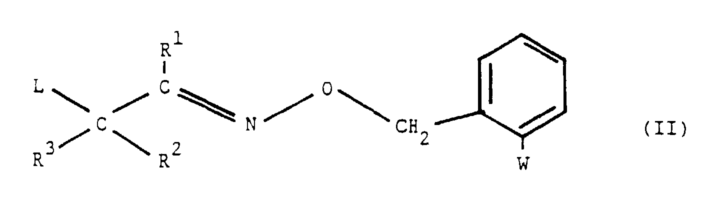 wherein W, L, R¹, R² and R³ are as defined in claim 1, the process comprising dehydroxylating a compound of formula (IV): 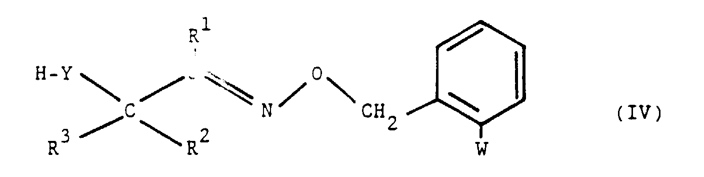 wherein Y is oxygen and R¹, R², R³ and W are as defined in claim 1.

3. A process for the preparation of a compound of formula (II): 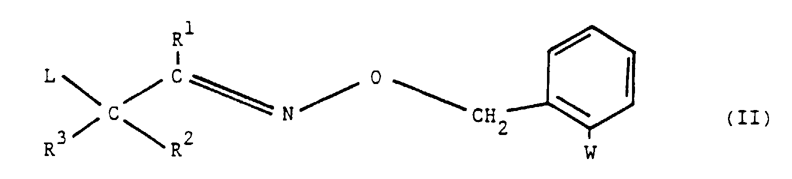 wherein R¹, R², R³ and W are as defined in claim 1 and L is chlorine, the process comprising reacting a compound of formula (V): 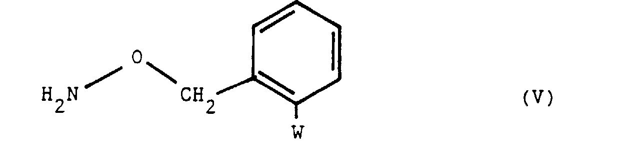 wherein W is as defined above, with a compound of formula (VII): 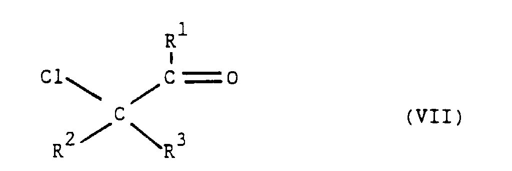 wherein R¹, R² and R³ are as defined in claim 1.

4. A process for the preparation of a compound of formula (I): 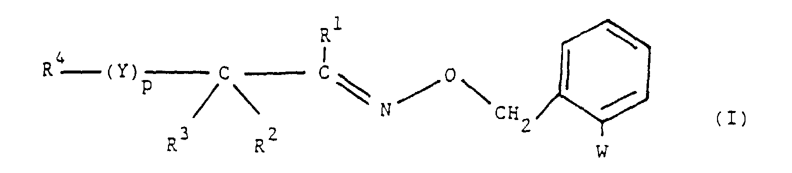 wherein Y is 0, S(O)ₙ or NR⁵, and W, p, R¹, R², R³, R⁴, R⁵ and n are as defined in claim 1, the process comprising reacting a compound of formula (IV) : 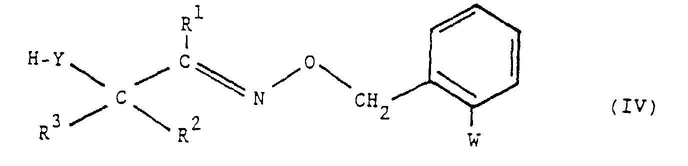 wherein Y is 0, S(O)ₙ or NR⁵, and W, R¹, R², R³, R⁵ and n are as defined in claim 1, with a compound of formula R⁴L, wherein R⁴ is as defined in claim 1 and L is a leaving group.

5. A compound of formula (II): 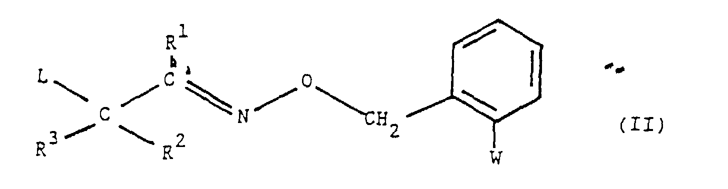 wherein W is CH₃O.CH=C.CO₂CH₃, CH₃O.CH=C.CONR⁶R⁷, CH₃O.N=C.CO₂CH₃ or CH₃O.N=C.CONR⁶R⁷; R¹ is hydrogen, C₁₋₄ alkyl, C₁₋₄ haloalkoxy or cyano; R², R³, R⁶ and R⁷ are independently hydrogen or C₁₋₄ alkyl; and L is a leaving group; provided that when L is chlorine, W is CH₃O.CH=C.CO₂CH₃, and R² and R³ are both hydrogen then R¹ is not methyl.

6. A compound of formula (IV): 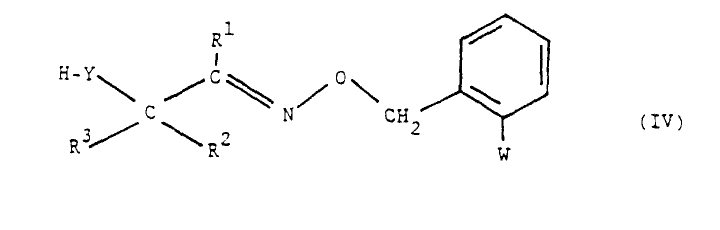 wherein Y is 0, S(O)ₙ (wherein n is 0, 1 or 2) or NR⁵; W is CH₃O.CH=C.CO₂CH₃, CH₃ O.CH=C.CONR⁶R⁷, CH₃O.N=C.CO₂CH₃ or CH₃O.N=C.CONR⁶R⁷; R⁷ is hydrogen, C₁₋₄ alkyl, C₁₋₄ haloalkoxy or cyano; and R², R³, R⁶ and R⁷ are independently hydrogen or C₁₋₄ alkyl.

## Patentansprüche

1. Verfahren zur Herstellung einer Verbindung der Formel (I): 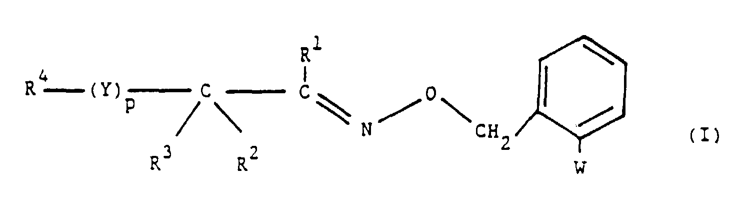 wobei Y O, S(O)ₙ (wobei n 0,1 oder 2 ist) oder NR⁵ bedeutet; W CH3O·CH=C·CO₂CH₃, CH₃O·CH=C·CONR⁶R⁷, CH₃O·N=C·CO₂CH₃ oder CH₃O·N=C·CONR⁶R⁷ bedeutet; R¹ ein Wasserstoffatom, eine C₁₋₄-Alkyl-, C₁₋₄-Halogenalkyl- oder Cyanogruppe bedeutet; R², R³, R⁵, R⁶ und R⁷ unabhängig voneinander ein Wasserstoffatom oder einen C₁₋₄-Alkylrest bedeuten; p 1 ist; und R⁴ ein Wasserstoffatom, einen C₁₋₁₀-Alkyl-, einen gegebenenfalls substituierten Aryl-, einen gegebenenfalls substituierten Heteroaryl-, einen gegebenenfalls substituierten Arylalkyl- oder einen gegebenenfalls substituierten Heteroarylalkylrest bedeutet; umfassend die Umsetzung einer Verbindung der Formel (II): 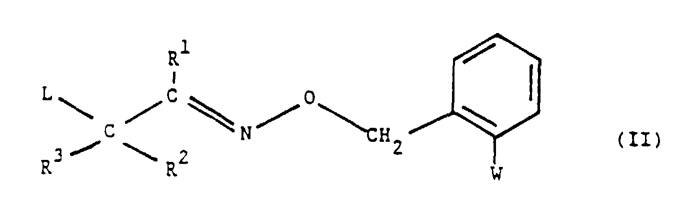 wobei W, R¹, R² und R³ die vorstehend angegebenen Bedeutungen besitzen und L eine Abgangsgruppe ist, mit einer Verbindung der Formel (III):
R⁴ ― Y―H (III)
wobei R⁴ und Y die vorstehend angegebenen Bedeutungen besitzen.

2. Verfahren zur Herstellung einer Verbindung der Formel (II): 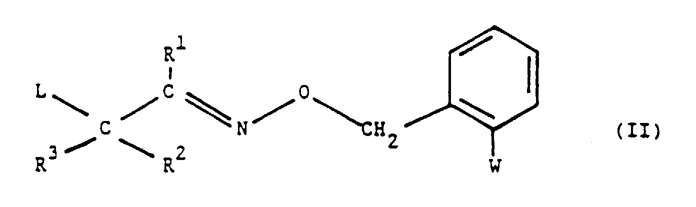 wobei W, L, R¹, R² und R³ die in Anspruch 1 angegebenen Bedeutungen besitzen, umfassend die Dehydroxylierung einer Verbindung der Formel (IV): 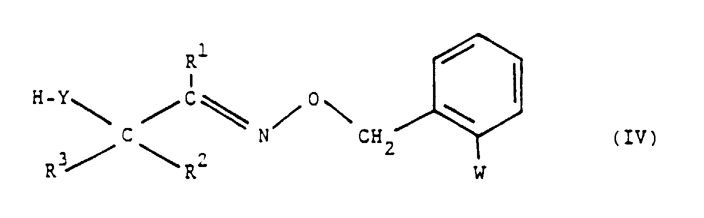 wobei Y ein Sauerstoffatom bedeutet, und R¹, R², R³ und W die in Anspruch 1 angegebenen Bedeutungen besitzen.

3. Verfahren zur Herstellung einer Verbindung der Formel (II): 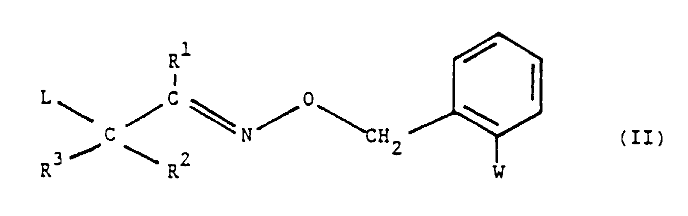 wobei R¹, R², R³ und W die in Anspruch 1 angegebenen Bedeutungen besitzen und L ein Chloratom bedeutet, umfassend die Umsetzung einer Verbindung der Formel (V): 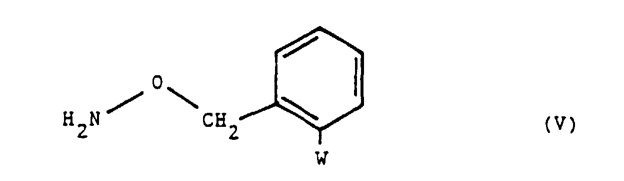 wobei W die vorstehend angegebene Bedeutung besitzt, mit einer Verbindung der Formel (VII): 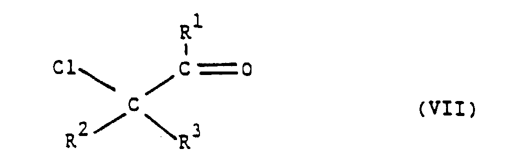 wobei R¹, R² und R³ die in Anspruch 1 angegebenen Bedeutungen besitzen.

4. Verfahren zur Herstellung einer Verbindung der Formel (I): 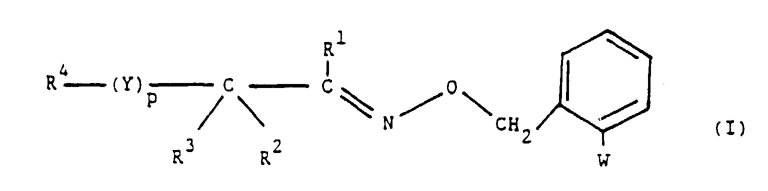 wobei Y O, S(O)ₙ oder NR⁵ bedeutet, und W, p, R¹, R², R³, R⁴, R⁵ und n die in Anspruch 1 angegebenen Bedeutungen besitzen, umfassend die Umsetzung einer Verbindung der Formel (IV): 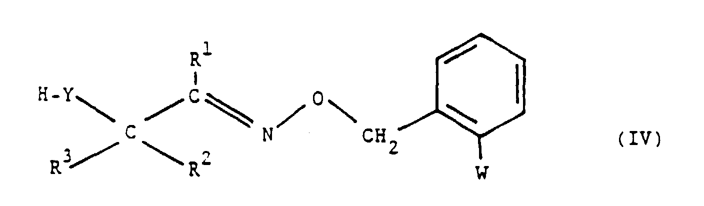 wobei Y O, S(O)ₙ oder NR⁵ bedeutet, und W, R¹, R², R³, R⁵ und n die in Anspruch 1 angegebenen Bedeutungen besitzen, mit einer Verbindung R⁴L, wobei R⁴ die in Anspruch 1 angegebene Bedeutung besitzt und L eine Abgangsgruppe ist.

5. Verbindung der Formel (II): 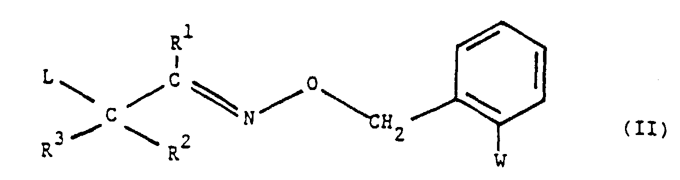 wobei W CH₃O·CH=C·CO₂CH₃, CH₃O·CH=C·CONR⁶R⁷, CH₃O·N=C·CO₂CH₃ oder CH₃O·N=C·CONR⁶R⁷ bedeutet; R¹ ein Wasserstoffatom, eine C₁₋₄-Alkyl-, C₁₋₄-Halogenalkoxy- oder Cyanogruppe bedeutet; R², R³, R⁶ und R⁷ unabhängig voneinander ein Wasserstoffatom oder einen C₁₋₄-Alkylrest bedeuten; und L eine Abgangsgruppe bedeutet; mit der Maßgabe, daß falls L ein Chloratom bedeutet, W CH₃O·CH=C·CO₂CH₃ ist, und falls R² und R³ beide ein Wasserstoffatom bedeuten R¹ keine Methylgruppe ist.

6. Verbindung der Formel (IV): 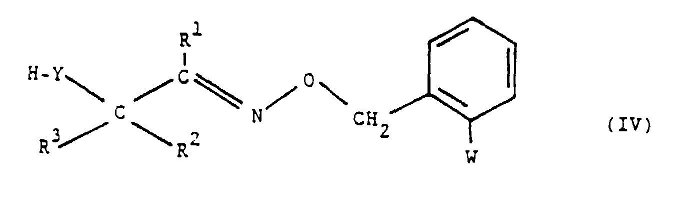 wobei Y O, S(O)ₙ (wobei n 0,1 oder 2 ist) oder NR⁵ bedeutet; W CH₃O·CH=C·CO₂CH₃, CH₃O·CH=C·CONR⁶R⁷, CH₃O·N=C·CO₂CH₃ oder CH₃O·N=C·CONR⁶R⁷ bedeutet; R¹ ein Wasserstoffatom, eine C₁₋₄-Alkyl-, C₁₋₄-Halogenalkoxy- oder Cyanogruppe bedeutet; und R², R³, R⁶ und R⁷ unabhängig voneinander ein Wasserstoffatom oder einen C₁₋₄-Alkylrest bedeuten.

## Revendications

1. Procédé de préparation d'un composé de Formule (I): 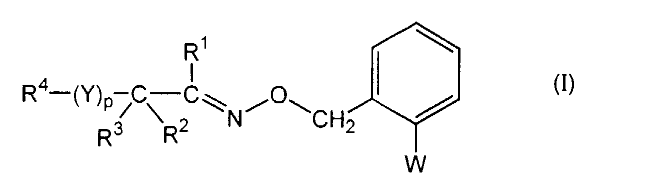 dans laquelle Y est O, S(O)ₙ (n étant égal à 0, 1 ou 2) ou NR⁵; W est CH₃O.CH=C.CO₂CH₃, CH₃O.CH=C.CONR⁶R⁷, CH₃O.N=C.CO₂CH₃ ou CH₃O.N=C.CONR⁶R⁷; R¹ est l'hydrogène, un groupe alkyle en C₁₋₄, un groupe haloalkyle en C₁₋₄ ou un groupe cyano; R², R³, R⁵, R⁶ et R⁷ sont indépendamment l'hydrogène ou un groupe alkyle en C₁₋₄; p est égal à 1; et R⁴ est l'hydrogène, un groupe alkyle en C₁₋₁₀, un groupe aryle éventuellement substitué, un groupe hétéroaryle éventuellement substitué, un groupe arylalkyle éventuellement substitué ou un groupe hétéroarylalkyle éventuellement substitué; le procédé comprenant la réaction d'un composé de Formule (II): 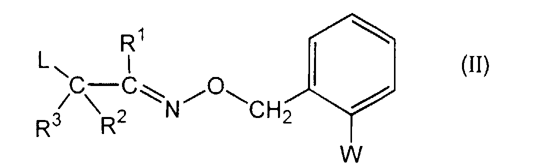 dans laquelle W, R¹, R² et R³ sont tels que définis précédemment et L est un groupe se séparant, avec un composé de Formule (III):
R⁴―Y―H (III)
dans la quelle R⁴ et Y sont tels que définis précédemment.

2. Procédé de préparation d'un composé de Formule (II): 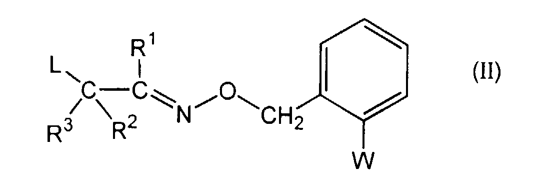 dans laquelle W, L, R¹, R² et R³ sont tels que définis dans la revendication 1, le procédé comprenant la déshydroxylation d'un composé de Formule (IV): 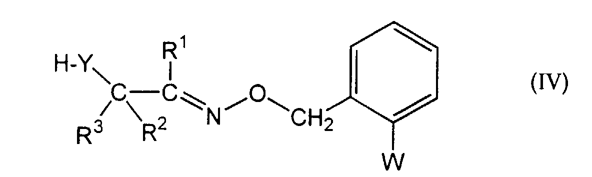 dans laquelle Y est l'oxygène et R¹, R², R³ et W sont tels que définis dans la revendication 1.

3. Procédé de préparation d'un composé de Formule (II): 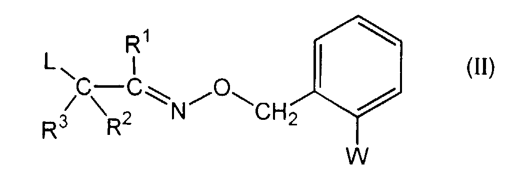 dans laquelle R¹, R², R³ et W sont tels que définis dans la revendication 1 et L est le chlore, le procédé comprenant la réaction d'un composé de Formule (V): 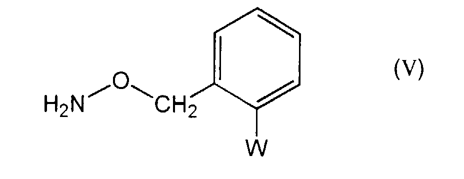 dans laquelle W est tel que défini ci-dessus, avec un composé de Formule (VII): 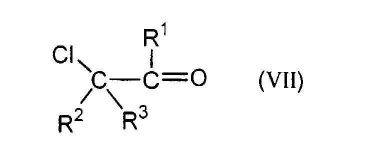 dans laquelle R¹, R² et R³ sont tels que définis dans la revendication 1.

4. Procédé de préparation d'un composé de Formule (I): 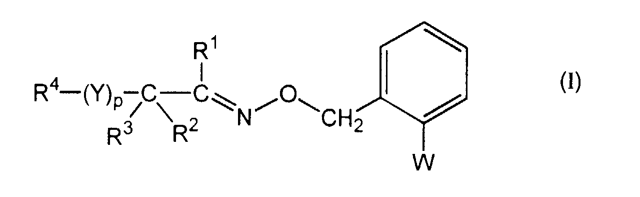 dans laquelle Y est O, S(O)ₙ ou NR⁵ et W, p, R¹, R², R³, R⁴, R⁵ et n sont tels que définis dans la revendication 1, le procédé comprenant la réaction d'un composé de Formule (IV): 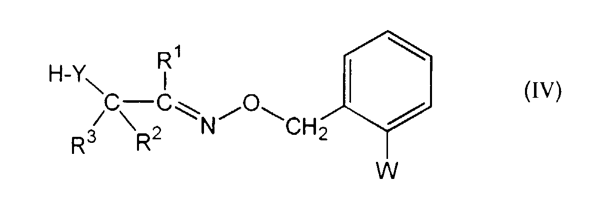 dans laquelle Y est O, S(O)ₙ ou NR⁵ et W, R¹, R², R³, R⁵ et n sont tels que définis dans la revendication 1, avec un composé de Formule R⁴L, dans laquelle R⁴ est tel que défini dans la revendication 1 et L est un groupe se séparant.

5. Composé de Formule (II): 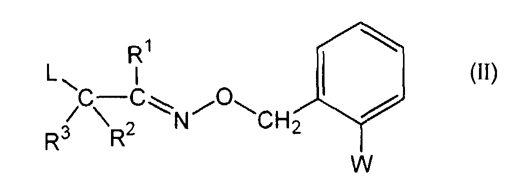 dans laquelle W est CH₃O.CH=C.CO₂CH₃, CH₃O.CH=C.CONR⁶R⁷, CH₃O.N=C.CO₂CH₃ ou CH₃O.N=C.CONR⁶R⁷; R¹ est l'hydrogène, un groupe alkyle en C₁₋₄, un groupe haloalcoxy en C₁₋₄ ou un groupe cyano; R², R³, R⁶ et R⁷ sont indépendamment l'hydrogène ou un groupe alkyle en C₁₋₄ et L est un groupe se séparant; à condition que quand L est le chlore, W est CH₃O.CH=C.CO₂CH₃, et R² et R³ sont tous deux l'hydrogène, R¹ ne soit pas un groupe méthyle.

6. Composé de Formule (IV): 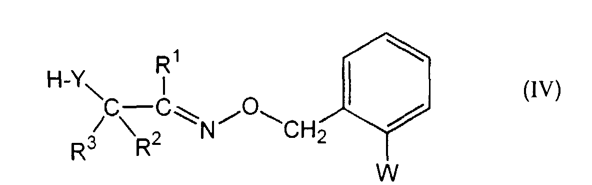 dans laquelle Y est O, S(O)ₙ (n étant égal à 0, 1 ou 2) ou NR⁵; W est CH₃O.CH=C.CO₂CH₃, CH₃O.CH=C.CONR⁶R⁷, CH₃O.N=C.CO₂CH₃ ou CH₃O.N=C.CONR⁶R⁷; R¹ est l'hydrogène, un groupe alkyle en C₁₋₄, un groupe haloalcoxy en C₁₋₄ ou un groupe cyano; R², R³, R⁶ et R⁷ sont indépendamment l'hydrogène ou un groupe alkyle en C₁₋₄.
